# EUROPEAN PATENT SPECIFICATION

(11) **EP 2 391 705 B1**
(45) Date of publication and mention of the grant of the patent: **22.04.2015**
(21) Application number: 10735435.9
(22) Date of filing: 29.01.2010
(51) Int. Cl.: C12M 1/00, A01G 33/00

(54) **METHOD AND APPARATUS FOR CULTIVATION OF ALGAE AND CYANOBACTERIA**
VERFAHREN UND VORRICHTUNG ZUR KULTIVIERUNG VON ALGEN UND CYANOBAKTERIEN
PROCÉDÉ ET APPAREIL POUR LA CULTURE D'ALGUES ET DE CYANOBACTÉRIES

(30) Priority: 30.01.2009 AU 2009900346; 24.08.2009 AU 2009904028
(43) Date of publication of application: 07.12.2011
(73) Proprietor: Zero Discharge PTY LTD, Prahran, Victoria 3181 (AU)
(72) Inventor: FALBER, Alexander, Bellevue Hill, New South Wales 2023 (AU)
(74) Representative: Potter Clarkson LLP
(86) International application number: PCT/AU2010/000090
(87) International publication number: WO 2010/085853

(56) References cited:
- WO-A1-99/20736
- WO-A1-2008/145719
- WO-A1-2009/043763
- WO-A2-2008/079724
- WO-A2-2008/079724
- WO-A2-2008/115058
- DE-A1- 10 322 111
- JP-A- 4 091 781
- US-A- 4 420 796
- US-A- 4 952 443
- US-A- 4 952 443
- US-A1- 2007 048 859
- US-A1- 2009 155 864
- US-A1- 2010 087 006
- US-B1- 6 287 852
- US-B1- 6 507 688
- US-B1- 6 509 188
- US-B1- 6 509 188
- DATABASE WPI Week 199219 Thomson Scientific, London, GB; AN 1992-154811 XP002716690, & JP H04 91781 A (HITACHI LTD) 25 March 1992 (1992-03-25)
- DATABASE WPI Week 200022 Thomson Scientific, London, GB; AN 2000-249659 XP002716691, & JP 2000 060533 A (MATSUSHITA DENKI SANGYO KK) 29 February 2000 (2000-02-29)
- DATABASE WPI 19 May 2010 Derwent Publications Ltd., London, GB; AN 1992-246276 & JP 4 166 017 A (ASAHI GLASS CO LTD) 06 November 1992
- DATABASE WPI 19 May 2010 Derwent Publications Ltd., London, GB; AN 1992-387560 & JP 4 287 678 A (HITACHI LTD) 13 October 1992
- DATABASE WPI 19 May 2010 Derwent Publications Ltd., London, GB; AN 2007-832560 & CN 1 982 433 A ((CHSC-N) CHINESE ACAD SCI PROCESS ENG RES INST) 20 June 2007

## Description

### FIELD

The invention relates to a bioreactor comprising a plurality of algae containment apparatuses and methods for growing algae or cyanobacteria related thereto.

### BACKGROUND

Production of biofuel from renewable processes is a key step towards securing a more responsible energy economy for the future. But beyond this, these fuels must be used in way that does not generate, or minimises, harmful emissions such as carbon dioxide.

Many important advances in biofuel production have been made. However, while advances in commercial production of algae has made great leaps regarding species selection, growth media, and even genetic manipulation, there remains a disproportionate lack of advance in the areas relating to working algal pond depth and harvesting. Algal pond depth is restricted by limited penetration of sunlight into the pond. Efficient harvesting of large quantities of algae requires large expenditures of energy and money.

Closed bioreactors are one means to overcome some of these disadvantages. However, such bioreactors are severely limited by cost and infrastructure requirements especially for biofuel applications. Open ponds are much more economic, particularly for cultivating algae, but are often is restricted by the availability of light to specific dimensions within the algal ponds. That is, light may only penetrate to a certain depth leaving many algae with sub-optimal illumination for growth, or even no light for growth.

In addition most algae growing systems, e.g. closed bioreactors or open ponds, experience photo- inhibition caused by over exposure to sunlight levels at the water surface. In an open pond this occurs a within the first few centimetres of water where little algae growth results. Only after the incoming sunlight intensity has been greatly reduced by the absorbance of photo-inhibited algae at the surface, do algae begin to grow at intermediate depths.

Furthermore, algae depend on photosynthetic pigments, such as chlorophyll, that selectively absorb certain wavelengths of light while other wavelengths are of lesser use, thus further reducing the overall utility of incoming solar radiation. As a result, algae systems exhibit low solar-photosynthetic efficiency. By most estimates, the photosynthetic efficiencies of current algae bioreactor systems average between 2% to 7% and have a maximum theoretical yield of 11% solar-photosynthetic efficiency.

Therefore, there is a need for an improved bioreactor that addresses the disadvantages of previous bioreactors with respect to bioreactor design, cost, and the inherent biological incompatibilities between the available light in such bioreactors and the algal photosynthetic system.

US 6,287,852 relates to a photosynthetic culture system that has a culture bath holding a fluid containing plant microorganism, carbon dioxide supplying means for supplying carbon dioxide to the fluid in the culture bath, light-conducting plate in the form of a flat plate placed oppositely to a light-receiving culture surface existing on the side of said culture bath, and a light-receiving panel mounted on the upper end surface of the light-conducting plate.

JP H04 91781 relates to a method for promoting photosynthesis by passing light of a wavelength band in a light source spectrum distribution of sunlight through a means for converting the light into a wavelengths band required for photosynthesis.

US 6,509,188 relates to a photobioreactor that has a reactor chamber that is made of light-transparent material and that has an increased surface area.

It is to be understood that any reference herein to prior art does not constitute an admission that such art forms a part of the common general knowledge in the art, in Australia or any other country.

### SUMMARY

A first aspect according to the claimed invention provides a bioreactor comprising a plurality of algae containment apparatuses for growing algae or cyanobacteria, comprising a luminescent material, wherein the luminescent material is suitable to absorb light of a first wavelength or wavelength range and emits visible light of a second wavelength or wavelength range, wherein the second wavelength or wavelength range is suitable for growing algae or cyanobacteria.

We provide use of the bioreactor of the invention, for growing algae or cyanobacteria.

An aspect provides a method for growing algae or cyanobacteria comprising growing the algae or cyanobacteria in he bioreactor of the invention.

We provide use of a luminescent material for growing algae or cyanobacteria, wherein the luminescent material is suitable to absorb light of a first wavelength or wavelength range and emit visible light of a second wavelength or wavelength range, wherein the second wavelength or wavelength range is suitable for growing algae or cyanobacteria.

We provide a method for growing algae or cyanobacteria comprising subjecting the algae or cyanobacteria to visible light produced by a luminescent material suitable to absorb light of a first wavelength or wavelength range and emit the visible light of a second wavelength or wavelength range, wherein the second wavelength or wavelength range is suitable for growing algae or cyanobacteria.

We provide an alga or a cyanobacterium when grown according to any one of the preceding uses or methods.

We provide a biofuel produced from the alga of the preceding aspect.

We provide a method for improving a bioreactor for growing algae or cyanobacteria comprising providing the bioreactor with a luminescent material suitable to absorb light of a first wavelength or wavelength range and emit visible light of a second wavelength or wavelength range, wherein the second wavelength or wavelength range is suitable for growing algae or cyanobacteria or providing the bioreactor with the algae containment apparatus of the first aspect.

### BRIEF DESCRIPTION OF THE DRAWINGS

Figure 1 provides absorbance spectra of chlorophylls and similar compounds.
Fig. 1a depicts absorbance spectra for chlorophyll a and chlorophyll b and a schematic absorbance spectrum for a luminescent material that absorbs green light. Also depicted is the corresponding schematic emission spectrum for the luminescent material illustrating emission of red light that overlaps with chlorophyll absorbance.
Fig. 1b depicts absorbance spectra corresponding to those illustrated in Fig. 1a, without the schematic absorbance and emission spectra of the luminescent material. Also illustrated are the absorbance spectra of β- carotene, phycoerythrin and phycocyanin. Phycoerythrin and phycocyanin are phycobilins that absorb light for photosynthesis in cyanobacteria.
Fig. 1c depicts absorbance spectra of chlorophyll a, chlorophyll b, and carotenoids (top) and the related photosynthetic action spectrum (bottom).
Figure 2 provides the chemical equations describing the process of carbon dioxide scrubbing by reaction with an amine (monoethanolamine, MEA) and the reversible reaction upon heating for subsequent release of carbon dioxide.
Figure 3 provides a schematic representation of one embodiment of a working algal bioreactor including a plurality of substrates comprising a luminescent material, whereby algae grow on the substrates and upon sunlight exposure the algae can continue to grow in darkness beyond the depth of penetrative sunlight in the bioreactor or during the night.
Figure 4 provides schematic representations of an embodiment of the algae containment apparatus and an embodiment of a bioreactor comprising the algae containment apparatus.
Fig. 4a is an orthogonal section of an open pond bioreactor comprising a plurality of the algae containment apparatuss.
Fig. 4b is a transverse cross section of an algae containment apparatus.
Fig. 4c depicts an overhead perspective view of an alternative raceway bioreactor comprising a plurality of algae containment apparatuss.
Fig. 4d illustrates the shading and luminescent effects when algae are grown in algae containment apparatuss in sunlight.
Fig. 4e is an orthogonal view of an algae containment apparatus comprising a light guide positioned on top of the algae containment apparatus.
Fig. 4f is an elevation view according to Figs 4d and 4f of a plurality of algae containment apparatuss each comprising a light guide. Also shown is orange light emitted from the edge of the light guides.
Fig. 4g provides longitudinal cross sections of embodiments of the light guide of Fig. 4e.
Figure 5 graphs to optical absorbance of pure water and shows how the absorbance increases by a factor of 10 between 500 nm (about 0.0005 cm⁻¹) to 600 nm (nearly 0.005 cm⁻¹). This translates to an absorbance of about 1 over a 2 m path length.
Figure 6 provides an orthogonal representation (Fig. 6a) and a cross-sectional representation through section 6b-6 (Fig. 6b) of a prismatic light guide.

### DETAILED DESCRIPTION

The shallow depths at which traditional open pond algal systems operate to allow sufficient exposure to sunlight require large areas to obtain adequate algal growth. Bioreactors require smaller areas and allow larger volumes of algal growth, but suffer from the problem of restricted availability of light at depth.

The present apparatus and methods overcome this limitation by increasing the solar efficiency of algae grown in the bioreactor. Thus, more algae may be grown in a smaller volume of growth medium. Furthermore, the depth of the bioreactor may be up to 10 times greater than current designs and therefore would require 10 times less land area. Most importantly, due to the optimised light spectrum in the system, algae will be able to grow at higher concentrations within the bioreactor. Additionally, fewer materials and less transparent tubing should be required compared with current bioreactors.

Thus, the present disclosure provides the following advantages:
- increased photosynthetic efficiency by conversion of light absorbed at a low level into light absorbed at a higher level;
- sustained algal or cyanobacterial growth at depths beyond that penetrable by a light source;
- improved efficiency of open systems towards the efficiency of closed systems;
- improved efficiency of closed systems;
- sustained algal or cyanobacterial growth during the night;
- reduced land use by replacement of ponds with bioreactors;
- utilisation of carbon dioxide from industrial and agricultural sources for photosynthesis; and
- ease of harvesting.

Algae or cyanobacteria grown according to the present disclosure can be used to produce biofuels, feeds, foods, food additives, bioactives such as pharmaceuticals and antibiotics, bioplastics, industrial chemicals or specialty chemicals.

In some embodiments, the luminescent material is disposed in or coated on a substrate. Thus, the substrate may emit light to the algae or cyanobacteria. In some embodiments, the substrate provides growing surfaces for the algae or cyanobacteria.

In one embodiment, the bioreactor comprises a substrate for providing growing surfaces for the algae or cyanobacteria. The substrate may comprise the luminescent material.

In some embodiments, the algae are grown in water or growth medium that is near, or in contact with, the luminescent material.

In one embodiment, the luminescent material may be part of alight guide that distributes light evenly down a water column in the shape of a sheet, tube, or other useful geometry where the algae are allowed to grow in a growth medium. The light guide functions according to methods and materials known in the art in a fashion optimised for the algae bioreactor.

In the embodiments of the aspects, the wavelength or wavelength range comprises visible light. In another embodiment, the first wavelength or wavelength range comprises green visible light and the second wavelength or wavelength range comprises red visible light. Typically, the first wavelength or wavelength range comprises about 500 nm to about 600 nm and the second wavelength or wavelength range comprises about 600 nm to about 700 nm.

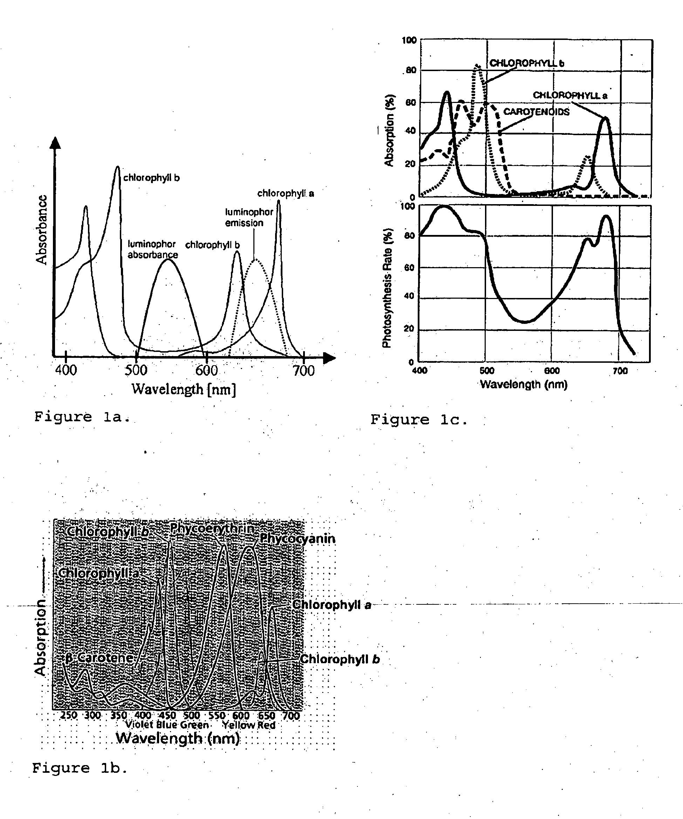

By most estimates, algae systems have a maximum theoretical yield of 11% solar-photosynthetic efficiency. Photosynthesis can provide up to 40% efficiency for conversion of solar energy to glucose at the wavelengths at which chlorophyll absorbs, i.e. 420 nm and 650 nm (Figure 1). At other wavelengths of the spectrum, however, photosynthesis is less than 1% efficient so that the whole spectrum averages between 5% to 10% efficiency. Up to 90% of all inefficiencies related to bioreactors for mass scale algae growth are related to incompatibility between the received solar radiation and the biologically optimal conditions for algae growth in terms of intensity and wavelength. The present disclosure addresses this incompatibility by promoting selective light absorbance through luminescent conversion of unused light to absorbable light, and thus may improve the solar efficiency of photosynthesis in a bioreactor or open pond.

The luminescent conversion of unused light to absorbable light may allow growth of algae or cyanobacteria at night or at depths at which light does not naturally penetrate.

In further embodiments of the aspects, the luminescent material comprises:
- a calcium sulfate phosphor; a zinc sulfate phosphor; a strontium aluminate phosphor; a calcium aluminate phosphor; a CaSrS phosphor; a CaS phosphor; or yttrium aluminium garnet (YAG, Y₃Al₅O₁₂), terbium aluminium garnet (TAG, Tb₃Al₅O₁₂), or Zex.

Alternatively, the luminescent material may comprise:
- a halogen-substituted, alkaline earth metal aluminate doped with at least one rare earth element activator, or
- a composition of: aL.bM.cAl.dSi.pP.O.:fR, wherein L is selected from Na and/or K; M is a divalent metal selected from one or more of the group consisting of Sr, Ca, Mg and Ba; Al, Si, P and O represent their respective elements; R is selected from one or more rare earth element activators; and wherein the variables a, b, c, d, p and f are: 0.0<a< 0.1; 0.0<b<0.3; 0.0<c<0.4; 0.0<d<0.3; 0.0<p<0.5; and 0.0<f≤0.25, with the proviso that at least one of the variables d and p is > 0, and at least one of the variables a and b is > 0.

Otherwise, the luminescent material may comprise:
- antimony-activated calcium fluorophosphate, lead-activated calcium tungstate, tin-activated strontium magnesium orthophosphate or manganese-activated magnesium fluorogermanate;
- yttrium and/ or gadolinium oxides activated by trivalent europium and having the formula

   (YₐGd₁₋ₐ)₂O₃: Eu³⁺, preferably a=1;
- calcium- and terbium-activated aluminates, silicates, phosphates and borates selected from compounds of the formulae
   CeMgAl₁₁O₁₉: Tb
   Y₂SiO₅: Ce, Tb
   LaPO₄: Ce, Tb
   LaMgB₅O1₀: Ce, Tb; or
- alkaline earth hexagonal aluminates of the B-alumina structure, or alkaline earth chlorophosphates, each activated by divalent europium and having the formula
   BaMgAl₁₀O₁₇: Eu²⁺
   BaMg_{1.7}Al₂₀O_{32.7}: Eu₂₊
   BaMg₂Al₂₄O₃₉: Eu₂₊
   (Sr,Ca,Ba)₁₀Cl₂(PO₄)₆: Eu²⁺.

According to some embodiments of the aspects, the luminescent material is disposed in or coated on a substrate. The substrate provides growing surfaces for the algae or cyanobacteria. The luminescent material may be disposed in or coated on one or more surfaces of the substrate. In certain embodiments, the substrate may comprise more than one luminescent material. If more than one substrate is present, the individual substrates may comprise different luminescent materials or the individual substrates may comprise more than one luminescent material. If more than one luminescent material is present, the luminescent materials may be separated or mixed.

In some embodiments, the substrate comprises a dielectric material. The dielectric material may comprise a polymer, glass, or quartz. The polymer may comprise acrylate or polycarbonate. In one embodiment, the polymer is polymethyl methacrylate. Quartz and certain polymers may be of benefit because they transmit UV light.

In one embodiment, the substrate is the luminescent material.

In one embodiment, the bioreactor includes a bank or storage cell comprising a further luminescent material for use in the bioreactor during the night or at depths not exposed to light. In one embodiment, such luminescent materials will have long glow times and provide low light levels necessary for heterotrophic, nocturnal growth of algae using any number of organic carbon sources as is known in the art.

In the embodiments, the luminescent material absorbs the first wavelength or wavelength range from sunlight. Alternatively, the luminescent material may absorb the first wavelength or wavelength range from an artificial light source, such as a fluorescent light.

In particular embodiments, the bioreactor utilises an industrial source of carbon dioxide. The industrial source of carbon dioxide may be post-combustion carbon dioxide, pre-combustion carbon dioxide, or flue gas, either treated or untreated. Post-combustion carbon dioxide may be isolated using a solvent, a membrane, a zeolite, or a cryogen. In some embodiments, the solvent is an amine. Typically, the solvent is monoethanolamine (MEA) or triethanolamine (TEA). In alternative embodiments, pre-combustion carbon dioxide may be isolated using integrated gasification combined cycle (IGCC). In some embodiments, IGCC produces hydrogen or syngas.

In another embodiment, the carbon dioxide is received as untreated flue gases and is absorbed by the algae growth medium kept at a basic pH where the algae take up inorganic carbon as anionic carbonates where algae species, such as *Coccolithophores,* known for their ability to take up calcium carbonates, are grown in the bioreactor or pond.

In another embodiment, the carbon dioxide is received from untreated flue gases from a power station utilising oxygen fired combustion where flue gases can be up to 95% carbon dioxide. Such systems may or may not use recycled flue gases to dilute the pure oxygen.

In other embodiments, the bioreactor utilises an agricultural source of carbon dioxide. For-example, dairy effluent wastewater may be treated in a bio-digester to generate biogas, typically a mixture of methane and carbon dioxide. Emissions from combustion of the biogas may be used as a source of carbon dioxide for photosynthesis as well as the carbon dioxide component of the biogas itself. Alternatively, human wastewater or sewage, either treated or untreated, may be utilised.

Persons skilled in the art will appreciate that suitable bioreactors, or bioreactors that may be suitably adapted, are known.

In some embodiments, the substrate may be filtered. Filtering enables isolation of the substrate for simple harvesting of the algae. In one embodiment, the algae or cyanobacteria may be harvested from the substrate. Harvesting may comprise wiping, scraping, washing, rinsing, drying or partially drying the algae or cyanobacteria, or altering the pH of the growth medium to disrupt binding of the algae.

In one embodiment, where algae are grown in water and are not bound to a surface, harvesting is accomplished by preliminary concentration of the algae followed by aggregation and filtration or centrifugation. Preliminary concentration can be accomplished using sonic standing waves in a controlled containment that causes algae to concentrate at the nodes of the standing waves. The columns of concentrated algae can be diverted or separated from water with a lower concentration of algae. This concentrate can then be aggregated, filtrated, or centrifuged as is known in the art.

As used in the subject specification, the singular forms "a", "an" and "the" include plural aspects unless the context clearly dictates otherwise. Thus, for example, reference to "a luminescent material" includes a single luminescent material, as well as two or more luminescent materials and so forth.

As used herein, "acrylate" refers to a polymer, homopolymer or copolymer, derived from the monomer acrylic acid or its derivatives possessing one or more functional groups. Derivatives include, for example, methyl acrylate, ethyl acrylate, methacrylate and methyl methacrylate.

As used herein, "alga" and "algae" refer to the singular and plural forms of the word, respectively. "Algae" lack the many distinct organs found in plants, but comprise a nucleus enclosed within a membrane and chloroplasts bound in one or more membranes. "Algae" are eukaryotic organisms that comprise chlorophyll that harvests sunlight for autotrophism via photosynthesis. Chlorophyll includes chlorophyll a, chlorophyll b, and chlorophyll c. Each of chlorophyll a, b, and c possess two major absorbance peaks in the electromagnetic spectrum: one near 420 nm and one near 650nm (blue light and red light, respectively; Figure 1). Chlorophyll has a lesser degree of absorbance of ultraviolet (UV), green, yellow, orange, or infrared light. "Algae" include Axodines, Bolidomonas, Brown algae, Charophyta, Cryptomonads, Diatoms, Dinoflagellates, Euglenids, Eustigmatophytes, Glaucophytes, Golden algae, Green algae, Heterokonts, Pelagophyceae, Phaetothamniophyceae, Pinguiophyceae, Prymnesiophyta, Raphidophytes, Red algae, Synurids, and Yellow-green algae. Microscopic algae and phytoplankton are particularly contemplated. Photosynthetic pigments in algae include chlorophyll *a* and chlorophyll *b*. Photosynthetic algae are relatively simple and cost-effective to grow and maintain. Algae can grow photosynthetically using carbon dioxide and sunlight, plus a minimum amount of trace nutrients. They also can alternatively or additionally grow heterotrophically on another carbon source, such as glucose or sucrose, or waste water. They are generally regarded as environmentally friendly and safe for human operators.

As used herein, "algae containment apparatus" refers to any container or vessel that supports a biologically active environment in which algae or cyanobacteria grow. An "algae containment apparatus" has no predetermined length other than that dictated by factors of practical engineering and includes tubes or tubular triangular shaped bags. An "algae containment apparatus" may be rigid or flexible. In one embodiment, an "algae containment apparatus" is a substrate.

As used herein, "bioreactor" refers to any device or system larger than an "algae containment apparatus" that supports a biologically active environment in which algae or cyanobacteria grow. A "bioreactor" may be an open system or a closed system. Thus, a "bioreactor" may include a pond, lake or raceway. Alternatively, a "bioreactor" may be a vessel. These vessel "bioreactors" are commonly cylindrical, ranging in size from litres to cubic meters, and are often made of stainless steel. Alternatively, a "bioreactor" may be made of concrete or a polymer. To be used as a bioreactor, a carbon source, an energy source and trace nutrients are required. For photosynthetic algae or cyanobacteria, a carbon source is carbon dioxide, and an energy source is light, typically sunlight.

In the claims that follow and in the description of the invention, except where the context requires otherwise due to express language or necessary implication, the word "comprise" or variations such as "comprises" or "comprising" is used in an inclusive sense, i.e. to specify the presence of the stated features, but not to preclude the presence or addition of further features in various embodiments of the invention.

As used herein, "cyanobacterium" and "cyanobacteria" refer to the singular and plural forms of the word, respectively. "Cyanobacteria" are prokaryotic, photosynthetic organisms that are also know as blue-green algae, but are not true algae. In contrast to algae that employ chlorophyll for photosynthesis, "cyanobacteria" generally employ phycobilins to absorb light for photosynthesis. Phycobilins in "cyanobacteria" include phycoerythrin, phycocyanin and allophycocyanin. Phycobilins predominantly absorb light in the green/ yellow/ orange (and near red) region of the electromagnetic spectrum from about 500 nm to about 650 nm, a region that chlorophyll absorbs very poorly.

The term "dielectric" refers to a non-conducting substance.

As used herein, the term "conduit" or "light conduit" is used interchangeably with the term "light guide" and refers to any material or construct of multiple materials, solids or liquids, designed to transport light using internal reflection or total internal reflection. This internal reflection is dependant upon the indices of refraction of the materials involved where the materials are transparent or translucent. Fibre optics are the most common example of conduits, but larger systems such as sheets and columns of any unspecified width or length are also included.

As used herein, "doped" or "doping" refers to the process of intentionally introducing impurities ("dopants") into a pure material to change its optical properties, i.e. with respect to luminescence, i.e. fluorescence or phosphorescence. "Dopants" include rare-earth elements.

As used herein, "emit" or "emission" refer to discharging or issuing light energy from a luminescent material.

As used herein, the term "excite" or "excitation" refers to causing change to, or exerting influence on, the luminescent material, particularly to the absorption of light energy by the luminescent material. "Exciting" refers to charging the luminescent material by absorption of light energy in readiness for its later emission as visible light of longer wavelength.

The term "fluorescent" or "fluorescence" and the term "phosphorescent" or "phosphorescence" each refers to a class of luminescence in which energy, usually light energy, is absorbed by a material at one wavelength or within a window of wavelengths and is subsequently emitted by the material as energy of a longer wavelength, or lower energy. "Fluorescence" can be distinguished generally from "phosphorescence" based on the time scale of light emission from the material, with phosphorescence emission being longer than fluorescence, but more accurately defined by the distinct pathways in the photophysics of relaxation from light induced excited states. These photophysical differences are clearly defined and known.

As used herein, "growth medium" refers to any mixture of trace nutrients capable of sustaining growth of algae or cyanobacteria. An example of a growth medium is a solution including the following constituents (the concentration for each constituent is mg/L): NaNO₃, 250; CaCl₂.2H₂O,25; MgSO₄.7H₂O,75; K₂HPO₄, 75; KH₂PO₄, 175; NaCl, 25; KOH, 31; FeSO₄.7H₂O, 5.0; H3BO₃, 11.4; ZnSO₄.7H₂O, 8.8 ; MnCl₂.4H₂O, 1.44; MoO₃, 0.7; CuSO₄.5H₂O, 1.57; Co[NO₃]₂.6H₂O, 0.50 and EDTA, 50. Alternatively, a growth medium may comprise wastewater or seawater, either treated or untreated.

The term "polymer" refers to a large molecule built up by repetitive bonding of many smaller units called monomers. The term "polymer" includes both homo-polymers comprising one species of constituent monomer and copolymers comprising more than one species of constituent monomer.

As used herein, "substrate" refers to any entity that provides a vehicle for the luminescent material and/or a growing surface for algae or cyanobacteria. Thus, in a vessel bioreactor for example, the substrate may include the walls and base of the bioreactor. In some embodiments, "substrate" refers to an entity distinct from the walls or base of the bioreactor that provides a vehicle for the luminescent material and/or growing surface for algae or cyanobacteria. In some embodiments, the algae containment apparatus or bioreactor is configured to prevent fouling of the substrate by direct contact with the algae. More than one "substrate" may be present in a bioreactor. In a particular embodiment, the "substrate" is the luminescent material.

The term "ultraviolet" or "UV" light refers to that portion of the solar electromagnetic spectrum subdivided as UVA, UVB, and UVC ranging from 400 to 100 nm.

The term "visible light" refers to that portion of the electromagnetic spectrum or photons visible to the naked eye, and is distinct from "ultraviolet" or "UV" light. This portion of the electromagnetic spectrum is typically regarded as falling between wavelengths of about 400 nm and about 750 nm.

Any luminescent material, which is capable of excitation by, or absorption of, light of a first wavelength or wavelength range and emission of visible light of a second wavelength or wavelength range longer than that of the first wavelength or wavelength range, can be used according to the present disclosure, provided that the emitted light is suitable for growing algae or cyanobacteria. In particular, light of the second wavelength or wavelength range should be absorbable by algae or cyanobacteria photosynthetic proteins or pigments, including chlorophyll a, chlorophyll b, chlorophyll c, and phycobilins such as phycoerythrin, phycocyanin and allophycocyanin.

The main characteristic driving selection of a luminescent material to be used is the emission wavelength of the material.

The luminescent material should be selected on the basis of the desire type of luminescence, i.e., fluorescence or phosphorescence.

Quantum efficiency may also be a characteristic driving selection of a luminescent material. Quantum efficiency should be of a magnitude sufficient to produce visible light suitable for photosynthesis.

The luminescent material should absorb light in the green region of the electromagnetic spectrum (about 500 nm to about 600 nm), a part of the spectrum largely unused by the chlorophyll pigments in algae. The luminescent material will absorb and use this energy to luminesce, or emit, red light between about 600 nm and about 700 nm, in one embodiment near 650 nm, which the algae can use extremely well for photosynthesis, and therefore growth.

In some embodiments, the luminescent material will absorb light from the UV and/or blue region, generally from about 250 nm to about 500 nm, and emit light in the green, yellow, orange or red region, generally from about 500 nm to about 650 nm, which may be then be absorbed by phycobilins of cyanobacteria and used for photosynthesis.

In one embodiment, more than one luminescent material may be present. For example, use of two luminescent materials, one that absorbs UV light and luminesces blue light and the other that absorbs green light and luminesces red light enables much more of the total solar spectrum to be used for photosynthesis in algae. Thus, the disclosure allows use of nearly all of the solar spectrum for photosynthesis, except for the infrared (IR) portion, although the IR portion may be used in cold climes for maintaining algal growth.

A luminescent material includes quite generally all inorganic, organic and organometallic materials capable of converting an input of absorbed photons into an output of photons of different energy, and the output comprises a visible light with a brightness and intensity sufficient for photosynthesis. The luminescent material may be coated on the substrate and/ or may be disposed in or contained in the substrate.

In one embodiment, the luminescent material may be any inorganic luminescent compound. In one embodiment, the inorganic luminescent compound may comprise a rare-earth doped inorganic crystal or a doped zinc sulphide. In another embodiment, the luminescent material may be any organic luminescent compound. In yet another embodiment, the luminescent material may comprise a quantum dot. In one embodiment, the luminescent material may be any organometallic luminescent compound.

The luminescent material may be, for example, a commercially available luminescent pigment or luminescent dye.

Examples of the luminescent (phosphorescent) material used include, but are not limited to, calcium sulfate phosphors (host crystal: CaS; activator: Bi); zinc sulfate phosphors (host crystal: ZnS; activator: Cu, e.g. "GSS" manufactured by Nemoto & Co., Ltd.); strontium aluminate or calcium aluminate phosphors (host crystal: strontium aluminate or calcium aluminate; activator: Eu, Dy, Nd, or the like; e.g. VGS-FAP or VGS3-FAP series manufactured by Visionglow International Pty Ltd; LumiNova^{®} G-300 series, BG-300 series, and V-300 series, manufactured by Nemoto & Co., Ltd.; "ULTRA GLOW series" NP-2810, NP-2820, and NP-2830, manufactured by Nichia Corporation; "R-bright" B and YG, manufactured by Lead Co., Ltd.; "Chemibright Powder" G-40-C, G-100-B, G-100-C, GB-80-B, and B-50-B, manufactured by Lumica Corporation); phosphors containing CaSrS, as a host crystal, and Bi, as an activator; and phosphors containing CaS, as a host crystal, and Eu or Tm, as an activator. Examples of suitable phosphorescent materials also include yttrium aluminium garnet (YAG, Y₃Al₅O₁₂), terbium aluminium garnet (TAG, Tb₃Al₅O₁₂), and Zex, which can emit a yellow light having a wavelength in the range of 530 to 590 nm.

Examples of the luminescent (fluorescent) material used include, but are not limited to, Rhodamine B, Rhodamine 6G, Rhodamine S, Eosine, Basic yellow HG, Brilliant sulfoflavine FF, Thioflavine, and Fluorescein. Examples of suitable fluorescent materials also include stilbene, benzooxazole, 9-oxo-xanthene, N-methyl-1,8-naphthyl-imide, 3-(4-chlorophenyl)pyrazoline, pyrazoline, imidazole, 1,2,4-triazole, oxazolidine-2-one, 1,8-naphthyl-imide, 4,4'-bis(2-methoxystyryl)-1,1'-biphenyl, 4,4'-bis(2-(1-pyrenyl)ethenyl)-1,1'-biphenyl, 4,4'-bis(2-(9-phenanthrenyl)ethenyl)-1,1'-biphenyl, 4,4'-bis(2-(9-anthracenyl)ethenyl)-1,1'-biphenyl, 4,4'-bis(2-(1-anthraquinonyl)ethenyl)-1,1'-biphenyl, 4,4'-bis{2-(2-fluorenyl)ethenyl}-1,1'-biphenyl, 1,4-bis(2-cyanostyryl)benzene, 1,4-bis(2-benzoxazoly)naphthalene, 2,5-bis(5-tertbutyl-2-benzoxazolyl)thiophene, 2,5-bis(2-benzoxazolyl)thiophene, 4,4-bis(benzoxazoyl)stilbene, 4,4'-bis(5-methyl-2-benzoxazolyl)stilbene, 1,2-bis(5-methyl-2-benzoxazolyl)ethylene, ethyl 5,6-benzocoumarin-3-carboxylate, 3-phenyl-5,6-benzocoumarin, N-methyl-4,5-diethoxy-1,8-naphthyl-imide, N-methyl-4-methoxy-1,8-naphthyl-imide, 3-(4-chlorophenyl)-1,5-diphenyl-2-pyrazoline, 3-(4-chlorophenyl)-1-phenyl-pyrazole, 4-methyl-7-diethylaminocoumarin, 1-(p-methanesulfonylphenyl)-3-(p-chlorophenyl)-2-pyrazoline, 1-(p-sulfonamidophenyl)-3-(p-chlorophenyl)-2-pyrazoline, pyrene, numerous derivatives of perylenes and any combination thereof. The fluorescent materials listed above can substantially completely absorb light over the entire UV, visible and near IR range, and subsequently re-emit it as longer wavelength light with very high brightness.

If the structure of the fluorescent material has the stilbene moiety, or the distyrylbiphenyl moiety, any chromophore groups, such as methoxyphenyl group, anthracene group, pyrene group, or 9,10-anthraquinone group, can be symmetrically bonded to such a stilbene moiety or distyrylbiphenyl moiety for enhancing brightness. Examples of such fluorescent materials include, but are not limited to, 4,4'-bis(2-methoxystyryl)biphenyl, 4,4'-bis2-(9-anthracenyl)ethylenyl}biphenyl, 4,4'-bis{2-(1-pyrenyl)ethylenyl}biphenyl, and 4,4'-bis{2-(1-anthraquinonyl)ethylenyl}biphenyl. When 4,4'-bis(2-methoxystyryl)biphenyl is used as the fluorescent material, it can be excited by UV light and subsequently emits a blue light having a wavelength between 450 nm and 490 nm. When 4,4'-bis{2-(9-anthracenyl)ethylenyl}biphenyl is used as the fluorescent material, it can be excited by UV light and subsequently emits a yellowish-green light having a wavelength between 520 nm and 550 nm. When 4,4'-bis{2-(1-pyrenyl)ethylenyl}biphenyl is used as the fluorescent material, it can be excited by UV light and subsequently emits a blue light having a wavelength between 450 nm and 490 nm. When 4,4'-bis{2-(1-anthraquinonyl)ethylenyl}biphenyl is used as the fluorescent material, it can be excited by UV light and subsequently emits a red light having a wavelength between 580 nm and 660 nm. In order to increase the brightness, a blue phosphor may be used with 4,4'-bis(2-methoxystyryl)biphenyl, or 4,4'-bis{2-(1-pyrenyl)ethylenyl}biphenyl to convert the emission to a blue light; a yellowish green phosphor may be used with 4,4'-bis{2-(9-anthracenyl)ethylenyl}biphenyl to convert the emission to a yellowish green light; and a red phosphor may be used with 4,4'-bis{2-(1-anthraquinonyl)ethylenyl}biphenyl to convert the emission to a red light.

The particle size of the luminescent material embedded in the substrate is not particularly limited, though it may comprise particles with an average diameter of 10 nm to 10 µm. If the luminescent material has an average particle diameter below 10 nm, it exhibits poor durability and significantly decreased brightness. If the luminescent material has an average particle diameter above 10 µm, greater visible light scattering arises. In the case of non-mineral luminescent materials that do not depend on a crystal lattice for their luminescent processes, as in most fluorescent dyes, the material can be fully dispersed or dissolved into the substrate as individual molecules absorbing light and subsequently luminescing.

Any lighting source that emits light of a wavelength that is suitable for energising the luminescent material may be used as an energy source. The source may be the sun, an incandescent device, a halogen device, or a fluorescent device. The device may be fluorescent such as bulb, globe or tube. In some embodiments, light is derived from sunlight.

The luminescent material may absorb light prior to algal absorption of light, since algal growth may impede absorbance of light by the luminescent material. The luminescent material may possess an emission period of sufficient duration to enable optimal algal absorption prior to abatement of the luminescence.

The luminescent material may be disposed in or coated on a substrate.

Algae or cyanobacteria may grow on a surface. The surface may be provided by the luminescent material. Alternatively, the surface may be provided by a substrate.

The substrate may be any shape that is amenable to exposure to light and provides a surface for growth of algae. Thus, shape selection will largely be driven by these considerations.

The substrate may be regular or irregular in shape. The dimensions may be equal or unequal and may or may not be in proportion. The substrate may be solid, hollow, porous, or laminar, for example. The substrate may comprise more than one material.

If more than one substrate is present, the substrates need not be homogenous; the substrates may include any combination of shapes or sizes or compositions of individual substrates.

The main characteristics driving selection of a substrate to be used according to the disclosure include high surface area, transmission of UV light, stable to sunlight or UV light, approximate neutral buoyancy in water, and durability.

The substrates may be arranged in close proximity to enable high concentrations of algae to be grown in relatively small volumes. The substrates may be arranged as closely packed layers or in an array. Alternatively, the substrates may not be in any fixed orientation and may circulate through the bioreactor randomly. The substrate may be a whole continuous surface or freely moving pieces of any range of sizes, shapes or composition. The substrates may be circulated by any appropriate mechanism that allows exposure to a fixed light source, such as sunlight or conduits, for example. Circulation may be achieved by bubbler, injector, pump, blade, impeller, jet, nozzle, stirrer, or shaker, for example.

The substrate may comprise a dielectric material. The dielectric material may comprise a polymer, glass, or quartz. In one embodiment, the polymer comprises acrylate or polycarbonate. In one embodiment, the polymer is polymethyl methacrylate or polycarbonate. In one embodiment, the polymer is polymethyl methacrylate.

In one embodiment, the luminescent material is disposed in a polymer. In another embodiment, the luminescent material is coated on a polymer substrate. The polymer may comprise: an acrylic, a urethane; an ester; a methacrylate; a thiophene; a co-polymer of any bond conjugated polymer; a light transparent polymer; a low ultra violet absorbent polymer; a heat conducting polymer; or an electrically conducting polymer. In another embodiment, the polymer may be: aniline based; pyrrole based; acetylene based; or furan based.

In another embodiment, the polymer may comprise polyurethane, polyester, polyamide, polyurea, polycarbonate and polymethyl methacrylate. The constituent monomers in the polymers of the present disclosure may be methacrylate-based, carbonate-based, acrylamide-based, methacrylamide-based, or styrene-based monomers.

Constituent monomers of the vinyl polymers that may be used include acrylic esters, specifically, e.g., methyl acrylate, ethyl acrylate, n-propyl acrylate, isopropyl acrylate, n-butyl acrylate, isobutyl acrylate, sec-butyl acrylate, tert-butyl acrylate, amyl acrylate, hexyl acrylate, 2-ethylhexyl acrylate, octyl acrylate, tert-octyl acrylate, 2-chloroethyl acrylate, 2-bromoethyl acrylate, 4-chlorobutyl acrylate, cyanoethyl acrylate, 2-acetoxyethyl acrylate, dimethylaminoethyl acrylate, benzyl acrylate, methoxybenzyl acrylate, 2-chlorocyclohexyl acrylate, cyclohexyl acrylate, furfuryl acrylate, tetrahydrofurfuryl acrylate, phenyl acrylate, 5-hydroxypentyl acrylate, 2-methoxyethyl acrylate, 3-methoxybutyl acrylate, 2-ethoxybutyl acrylate, 2-ethoxyethyl acrylate, 2-isopropoxy acrylate, 2-butoxyethyl acrylate, 2-(2-methoxyethoxy)ethyl acrylate, 2-(2-methoxyethoxy)ethyl acrylate, 2-(2-butoxyethoxy) ethyl acrylate, ω-methoxypolyethylene glycol acrylate (addition mol number: 9), 1-bromo-2-methoxyethyl acrylate, and 1,1-dichloro-2-ethoxyethyl acrylate.

In addition, the following monomers can be used. Methacrylic esters, specifically, e.g., methyl methacrylate, ethyl methacrylate, n-propyl methacrylate, isopropyl methacrylate, n-butyl methacrylate, isobutyl methacrylate, sec-butylmethacrylate, tert-butylmethacrylate, amylmethacrylate, hexylmethacrylate, cyclohexylmethacrylate, benzyl methacrylate, chlorobenzyl methacrylate, octyl methacrylate, stearylmethacrylate, sulfopropylmethacrylate, N-ethyl-N-phenylaminoethyl methacrylate, 2-(3-phenylpropyloxy)ethyl methacrylate, dimethylaminophenoxyethyl methacrylate, furfuryl methacrylate, tetrahydrofurfuryl methacrylate, phenyl methacrylate, cresyl methacrylate, naphthyl methacrylate, 2-hydroxyethyl methacrylate, 4-hydroxybutyl methacrylate, triethylene glycol monomethacrylate, dipropylene glycol monomethacrylate, 2-methoxyethyl methacrylate, 3-methoxybutyl methacrylate, 2-acetoxyethyl methacrylate, 2-acetoacetoxyethyl methacrylate, 2-ethoxyethyl methacrylate, 2-isopropoxyethyl methacrylate, 2butoxyethyl methacrylate, 2-(2-methoxyethoxy)ethyl methacrylate, 2-(2-ethoxyethoxy)ethyl methacrylate, 2-(2-butoxyethoxy)ethyl methacrylate, ω-methoxypolyethylene glycol methacrylate (addition mol number: 6), acryl methacrylate, and methacrylic acid dimethylaminoethylmethyl chloride salt can be exemplified.

Vinylesters, specifically, e.g., vinylacetate, vinyl propionate, vinyl butyrate, vinyl isobutyrate, vinyl caproate, vinyl chloroacetate, vinylmethoxy acetate, vinylphenyl acetate, vinyl benzoate and vinyl salicylate can be exemplified.

Acrylamides, e.g., acrylamide, methylacrylamide, ethylacrylamide, propylacrylamide, isopropylacrylamide, n-butylacrylamide, sec-butylacrylamide, tert-butylacrylamide, cyclohexylacrylamide, benzylacrylamide, hydroxymethylacrylamide, methoxyethylacrylamide, dimethylaminoethylacrylamide, phenylacrylamide, dimethylacrylamide, diethylacrylamide, β-cyanoethylacrylamide, N-(2-acetoacetoxyethyl)acrylamide, and diacetoneacrylamide can be exemplified.

Methacrylamides, e.g., methacrylamide, methylmethacrylamide, ethylmethacrylamide, propylmethacrylamide, isopropylmethacrylamide, n-butylmethacrylamide, sec-butylmethacrylamide, tert-butylmethacrylamide, cyclohexylmethacrylamide, benzylmethacrylamide, hydroxymethacrylamide, chlorobenzylmethacrylamide, octylmethacrylamide, stearylmethacrylamide, sulfopropylmethacrylamide, N-ethyl-N-phenylaminoethylmethacrylamide, 2-(3-phenylpropyloxy)ethylmethacrylamide, dimethylaminophenoxyethylmethacrylamide, furfurylmethacrylamide, tetrahydrofurfurylmethacrylamide, phenylmethacrylamide, cresylmethacrylamide, naphthylmethacrylamide, 2-hydroxyethylmethacrylamide, 4-hydroxybutylmethacrylamide, triethylene glycol monomethacrylamide, dipropylene glycol monomethacrylamide, 2-methoxyethylmethacrylamide, 3-methoxybutylmethacrylamide, 2-acetoxyethylmethacrylamide, 2-acetoacetoxyethylmethacrylamide, 2-ethoxyethylmethacrylamide, 2-isopropoxyethylmethacrylamide, 2-butoxyethylmethacrylamide, 2-(2-methoxyethoxy) ethylmethacrylamide, 2-(2-ethoxyethoxy) ethylmethacrylamide, 2-(2-butoxyethoxy) ethylmethacrylamide, ω-methoxypolyethylene glycol methacrylamide (addition mol number: 6), acrylmethacrylamide, dimethylaminomethacrylamide, diethylaminomethacrylamide, B-cyanoethylmethacrylamide, and N-(2-acetoacetoxyethyl)methacrylamide can be exemplified.

Olefins, e.g., dicyclopentadiene, ethylene, propylene, 1-butene, 1-pentene, vinyl chloride, vinylidene chloride, isoprene, chloroprene, butadiene, and 2,3-dimethylbutadiene can be exemplified.

Styrenes, e.g., styrene, methylstyrene, dimethylstyrene, trimethylstyrene, ethylstyrene, isopropylstyrene, chloromethylstyrene, methoxystyrene, acetoxystyrene, chlorostyrene, dichlorostyrene, bromostyrene, and vinylbenzoic acid methyl ester can be exemplified.

Vinyl ethers, e.g., methylvinyl ether, butylvinyl ether, hexylvinyl ether, methoxyethylvinyl ether and dimethylaminoethylvinyl ether can be exemplified.

As other examples, e.g., butyl crotonate, hexyl crotonate, dibutyl itaconate, dimethyl maleate, dibutyl maleate, dimethyl fumarate, dibutyl fumarate, methyl vinyl ketone, phenyl vinyl ketone, methoxyethyl vinyl ketone, glycidyl acrylate, glycidyl methacrylate, N-vinyloxazolidone, N-vinylpyrrolidone, acrylonitrile, methacrylonitrile, methylene moronnitrile, and vinylidene can be exemplified.

Two or more monomers may be used as co-monomers with each other according to purposes (e.g., improvement of hardness, flexibility, tensile strength and light fastness), thereby producing co-polymers.

The methods of adding and mixing the components with a polymer are not restricted. For example, methods of thoroughly mixing the powders, flakes or pellets of translucent polymers with the above components and then melt-mixing by an extruder may be used. In translucent thermoplastic polymers, methods of adding the above components to unhardened liquid state starting materials and thoroughly mixing and dispersing may be used. At this time, generally used additives, e.g., a thermal stabiliser, an antioxidant, a mould releasing agent, an antistatic agent, and a flame retarder may be added. Moulding may be performed according to ordinary methods. That is, in the case of thermoplastic polymers, covered pipes can be produced by a melt-extrusion method, shrink tubes can be produced by stretching and quenching of the pipes obtained by melt-extrusion, and covers can be produced by injection moulding, extrusion moulding and, if necessary, vacuum moulding. In the case of thermo-setting polymers, cast moulding is advantageous.

### Bioreactor Design

In one embodiment, sunlight can be introduced into the bioreactor by collection, diversion of IR light, and distribution using a parabolic dish, a prism, and light conduits, respectively. Parabolic dishes ensure efficient collection of solar energy without loss by reflection that occurs commonly off the surface of water in open pond systems. This arrangement should ensure the most efficient collection of solar energy without loss by reflection that occurs commonly off the surface of water in open pond systems. Once diverted, the IR portion of the collected energy can be used as a source of solar thermal energy.

In one embodiment, the solar collecting dish can be fitted with UV and IR filters to exclude damaging UV and heating radiation. However, if it is desired to raise the temperature of the medium in which the algae are growing, the IR filter may be removed to harness the IR radiation until the water is at the optimal temperature for growth of the algae.

Alternatively, a Fresnel lens, a mirror or a combination of mirrors can be used to collect light. Fresnel lenses are, at their simplest, glass or plastic sheets with finely scored lines or ridges formed in the sheets. They can be flat or formed with a curvature to provide greater focus into the bioreactor. Also contemplated is a bank or storage cell comprising a further source of luminescent material that can be charged during daylight hours and used during the night to sustain a low level of algae growth.

After collection and diversion of IR energy, light may be distributed using conduits known in the art. Such conduits allow deeper penetration of light into the bioreactor and increase the surface area from which light is emitted for photosynthesis.

A light conduit or light guide may be solid or hollow. A solid light conduit may include an optical fibre. A hollow light conduit may be filled with a liquid. Liquid-filled light conduits are known in the art, for example as disclosed in United States Patent 4,927,231, United States Patent 5,857,052, and United States Patent 6,507,688.

A light conduit known in the art may be scaled up for use in the bioreactor.

A light conduit known in the art may be modified, for example, to distribute light along its length in addition to transporting light from one end of the conduit to the other. For example, a translucent light guide may be etched to aid diffusion of light from the light conduit. In addition, the light guide may comprise a diffusing device, for example as disclosed in United States Patent 4,420,796.

A light conduit, for example a liquid-filled light guide may comprise a double wall. A liquid-filled lightguide may comprise a plastic outer wall and a plastic inner wall, the walls being spaced apart and filled with air, vacuum, or any number of transparent materials of a lower index of refraction than the inner liquid. Alternatively, the cavity formed between the spaced-apart walls may be filled with a gas, for example an inert gas such as nitrogen, carbon dioxide or argon as found in United States Patent 4420796. In such a light conduit, the air or other gas provides an appropriate refractive index to guide light in the conduit.

A liquid used in the light conduit may comprise any liquid suitable for conducting light, such as water, dimethylsulfoxide or ethylene glycol. The liquid may comprise a salt solution.

The light guide or light conduit may be angled top make best use of seasonal solar incident light. For example, the light conduit may be angled at about 70° relative to the ground to ensure internal reflection during winter at latitudes near 37° North or South where the indices of refraction of water, n=1.33, and air, n=~1, define the system's ability to transport sunlight in the conduit and the lowest solar angle is approximately 30° relative to the horizon.

In one embodiment, the surface area of the light collecting dish is compared to the surface area of the light conduit. For example, if the area of sunlight captured by a solar collector is 7m², the internal surface area of the light conduit may be measured as the internal circumference of the conduit multiplied by its length; for example, a light conduit 0.25 m in diameter and 9 m long will have a surface area of approximately 7 m², similar to the collecting dish above. Thus, a light conduit of these measurements may emit light along its length at an equal light intensity as the sunlight incident on the dish neglecting losses in the process of reflection. In another example, a light conduit 0.25 m in diameter and 18 m in length will emit light along its length at about half the light intensity of the sunlight incident on the collecting dish neglecting losses in the process of reflection.

Optimal growth rates for algae occur between 200 to 600 ft-c or 2,000 to 6,000 lux (1 ft-c = 10 lux) and growth decreases with higher or lower light intensities. Strong midday sunlight is around 100,000 lux with a lower end of 32,000 lux. The light intensities of the sun are about 5 to 17 times greater than necessary for maximal algae growth. This means that the surface area of the light conduit in one embodiment may be much greater than the area of the light collecting dish. However, in practice, the intensity of light in the conduit may fall off sharply moving from the surface of the light conduit towards the inner wall of the bioreactor. In practice, a light conduit that has at least 3 to 4 times the surface area of the collecting dish should emit sufficient light for optimal algae growth. Thus, relative to the embodiment above, the light conduit may be 0.35 m in diameter and 30 m in length.

In another embodiment, the light guide is a large sheet. For example a 7 m² collector dish can connected to a light guide sheet that is 4 m by 3.5 m and emit light evenly on both sides for a total surface area of 28m² and would thus be four times the surface area of the collector dish.

In another embodiment, the light guide is of the design developed by 3M^{™} and others known as a "prismatic light guide". This type of light guide has the advantage of being made within a large range of widths where the internal space of the light guide is filled with air, gas, or vacuum. The external walls are comprised of a material with a high index of refraction such as any plastic, polymer, or liquid as those described herein for general light guides. The external walls are comprised of a ridged linear pattern that creates a 90° prismatic shape that is sometimes altered to smaller angles for different light transport effects.

In one embodiment, the bioreactor will have a smooth, reflective inner surface or lining. The reflective lining may be white and also the lining may be plastic. The reflective lining may maximise the light available for algae growth by reflecting light that hits the inner wall of the bioreactor.

Exposure to light, typically sunlight, of the bank or storage cell during the day in the absence of algae, i.e. external to the bioreactor, will effectively "charge" the bank or storage cell so that it may be added to the bioreactor to provide light for continued photosynthesis to sustain a low level of algae growth during the dark hours. During the dark hours, algae perform respiration which depletes the overall biomass. The use of further luminescent materials during the night can offset some of these losses and may provide a healthy population of algae for the next batch.

Furthermore, the growth medium depth may be much deeper than the depth of sunlight penetration. Thus, a bank or storage cell may be used at depth as well as at night. As the algae-bound substrates near the surface, sunlight will be absorbed by both the algae and luminescent material, provided that the surface of the substrate is not entirely covered by algae. At greater depths, where no sunlight is present, the luminescent material will emit light for photosynthesis and therefore continuously feed light to the algae at the wavelength most necessary for photosynthesis. The substrates may be able to feed light to the algae for a number of hours.

When exposed to air, the growth medium is exposed to air at its surface, and a portion of carbon dioxide from the air is dissolved in the growth medium. Turning or mixing the growth medium increases exposure of the growth medium to air and enhances dissolution of carbon dioxide into the growth medium. Other methods of introducing carbon dioxide into the growth medium can be employed, as will be appreciated by those skilled in the art, including a carbon dioxide bubbler or jet (or multiple bubblers or jets), introducing carbon dioxide gas into the growth medium at one or multiple gas ports in the bioreactor. Carbon dioxide bubblers or jets can also serve as mixing and impelling means. Additionally, another method involves increasing the concentration of carbon dioxide in the air above the bioreactor, such as by forming a sealed enclosure over the bioreactor, and introducing carbon dioxide gas in the area over the surface of the growth medium and within the enclosure.

### Harvesting

Algae possess a strong ability to bind surfaces. In one embodiment, the algae will grow upon the surface of the luminescent material. In another embodiment, the algae will grow on the surface of the substrate. An advantage of growing algae on a surface is that the biomass presents itself in a concentrated state as a thick layer on the surfaces.

When more than one substrate is present, the substrates can be easily filtered, and therefore makes harvesting facile. In one embodiment, the filtered substrates covered with algae can be removed, washed with water to remove salts from the medium and the algae can then be removed from the substrate by a number of methods, one of which includes altering the pH of the growth medium to disrupt the binding of the algae to the substrate, or first drying the substrates and removing the algae as a powder. High pressure water jets may also be used to remove algae from the substrate. The substrates, now free of algae, can be returned to the system to support another batch of algae.

Harvesting may be accomplished by mechanical wiping, high pressure water, or allowing the algae to dry on the sheets to be collected as a semi-dry solid, for example. Harvesting may comprise wiping, scraping, washing, rinsing, drying, partially drying, shaking, vibrating, or altering the pH.

In one embodiment, the bioreactor will grow algae on substrates that allow algae to be harvested as a thick mass with limited water content thereby avoiding costly filtration and centrifuge processes. By removing only the amount of water necessary for utilising the algal biomass as a feedstock for direct gasification, for example, energy expenditures used in producing biofuel from the algal biomass will be minimised.

### Sources of Carbon

When used as a carbon source, carbon dioxide may be obtained from the ambient atmosphere. Alternatively, carbon dioxide may be derived from a specific source. These include industrial, agricultural and human sources.

### Industrial - PCC and IGCC

The present disclosure may be compatible with carbon dioxide capture methods that are currently being developed for clean coal technologies. These technologies mainly include Post Combustion Capture (PCC) using amine solvents and Integrated Gasification Combined Cycle process (IGCC) to produce hydrogen as a major component of "syngas".

These clean coal technologies are crucial in isolating the carbon dioxide component of flue gas emissions, but they do not offer a method of sequestering or professing the captured carbon dioxide *per se*. Importantly, IGCC offers a method of much higher thermodynamic efficiency in burning fuel, and would make the best use from the energy stored in algal biomass.

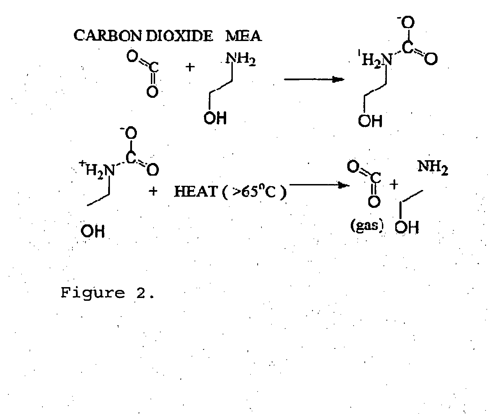

Post combustion capture of carbon dioxide is largely accomplished using amine solutions, typically monoethanolamine (MEA), that quantitatively react with all carbon dioxide from a relatively cool gas stream. The reaction is then reversed by heating the amine solution thereby releasing the carbon dioxide as a pure stream, particularly for sequestration. The chemical reaction is provided in Figure 2.

The capture of carbon dioxide by amines is a convenient way of handling large, concentrated volumes of carbon dioxide at standard pressures in aqueous solutions. Carbon dioxide normally has a relatively low solubility in water, especially in warm water (30°C). There are many amines that can be used for this process such as triethanolamine (TEA). The amines are largely reusable, biodegradable, and inexpensive. Contemplated herein is use of an amine that is non-toxic to algae in a growth medium to allow high concentrations of carbon dioxide injection into the bioreactor for providing a convenient source of inorganic carbon for the algae. Such a medium would be both a carbon dioxide sequester and an algal biomass generator. The algae may uptake carbon dioxide from the amine solvent.

Thus, the present disclosure will produce an algae biomass that can be used as a feedstock for gasification, thereby replacing a portion of the coal necessary for power production. In addition, the use of algal feedstock in IGCC will allow the large scale production of hydrogen gas, the most environmentally friendly fuel on earth, from a renewable resource.

### Industrial Untreated Flue Gas

Using untreated flue gas emissions as a source of carbon dioxide and hence inorganic carbon for algae growth has been hindered by the large amount of acidic sulfurous and nitrogenous components, solid particulates, and most importantly, the excess heat from flue gas which is commonly found in temperatures close to or greater than 80°C. To date, these temperatures and contaminants have precluded the use of flue gas emission without pre-treatment, though flue gas emissions are one of the very few viable sources of large quantities of concentrated carbon dioxide for mass scale algae production.

### Agricultural

It is envisaged that effluent waste, for example dairy effluent wastewater, may be treated in a bio-digester to generate biogas as a fuel. Methane may be produced from the bio-digestion of effluent wastewater by methanogenic bacteria. Furthermore, emissions from combustion of the biogas may be used as source of carbon dioxide for photosynthesis. The resulting algae biomass may be used as a biofuel feedstock or a protein rich feed for livestock.

Subsequently, the treated effluent waters may be used as growth medium comprising trace nutrients for growth. Finally, the water depleted of nutrients via algal growth, can be distributed as irrigation for the farm pastures.

Alternatively, human wastewater or sewage may be used.

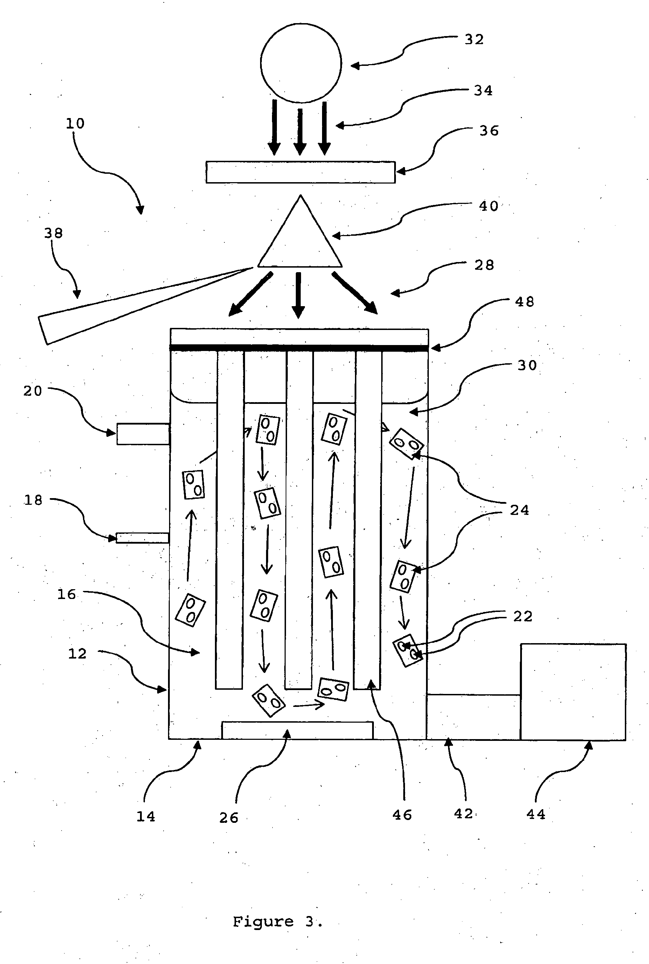

In one embodiment depicted in Figure 3, bioreactor 10 comprises a circular vessel bioreactor having wall 12 and base 14 for holding growth medium 16. Bioreactor 10 comprises inlet 20, which allows introduction of fresh growth medium 16 and starter algae (not depicted) into bioreactor 10. Carbon dioxide is introduced to bioreactor 10 via inlet 20. Algae 22 grow on the surface of a plurality of substrates 24. Substrates 24 comprise a luminescent material (not depicted). Substrates 24 are circulated through bioreactor 10 by mixer 26. At intermittent intervals, algae 22 growing on substrate 24 are exposed to light 28 near the upper surface 30 of growth medium 16, thereby enabling the luminescent material to absorb light energy for later emission to algae 22. Light 28 is derived from the sun 32 and sunlight 34 is collected using parabolic dish 36. Infrared radiation 38 is diverted using prism 40. At a predefined time, algae 22 growing on substrate 24 are removed from bioreactor 10 by outlet 42 and delivered to filter 44 for separation of algae 22 plus substrate 24 from growth medium 16 for subsequent harvest of algae 22 from substrate 24. In one embodiment, light 28 is distributed via conduits 46 arranged on support 48 within bioreactor 10 to enable further exposure of the luminescent material and algae 22 to light at depths of growth medium 16 otherwise impenetrable to light 28.

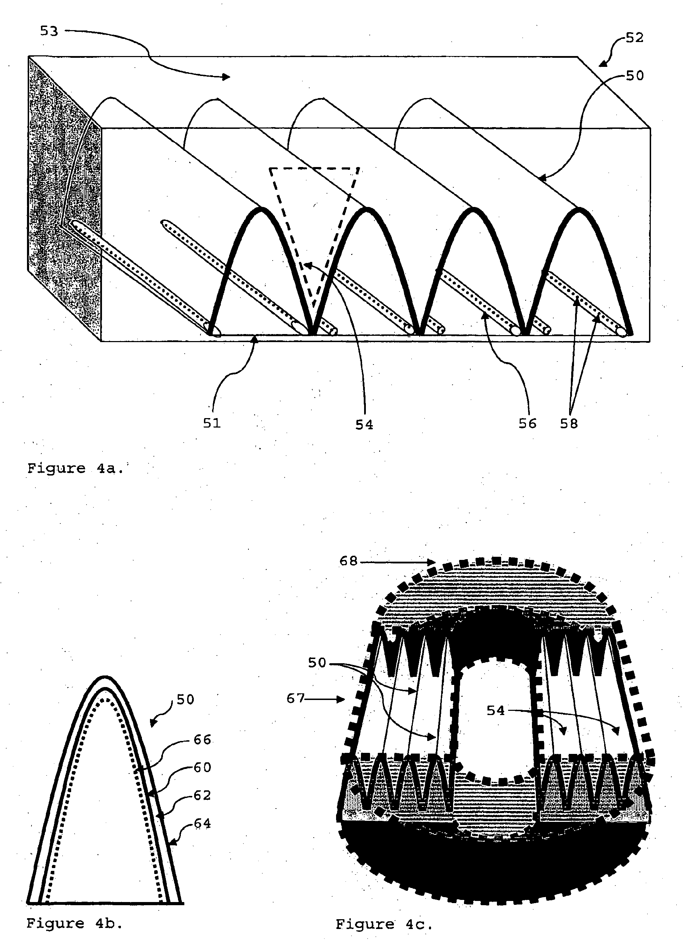

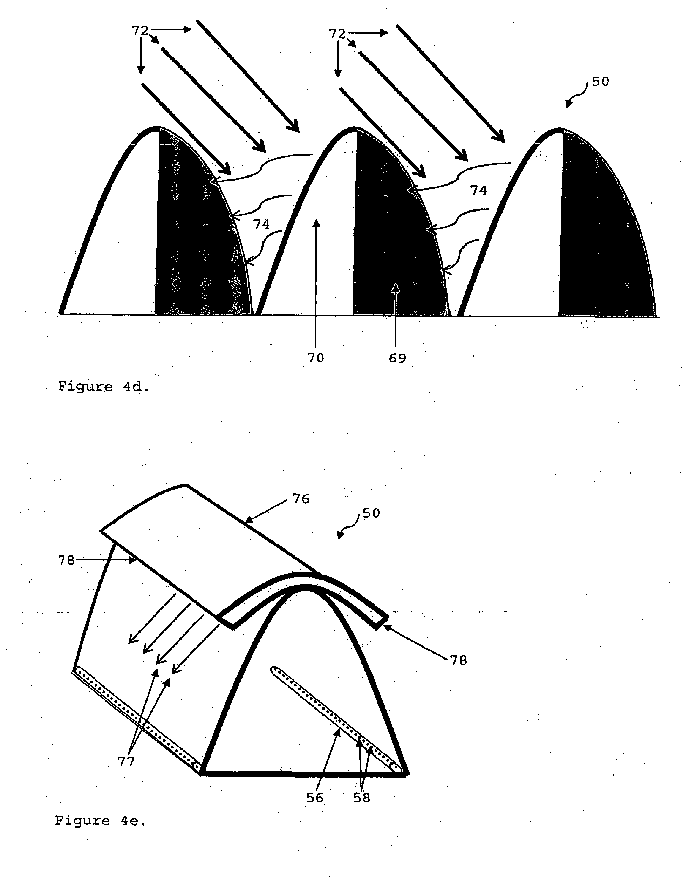

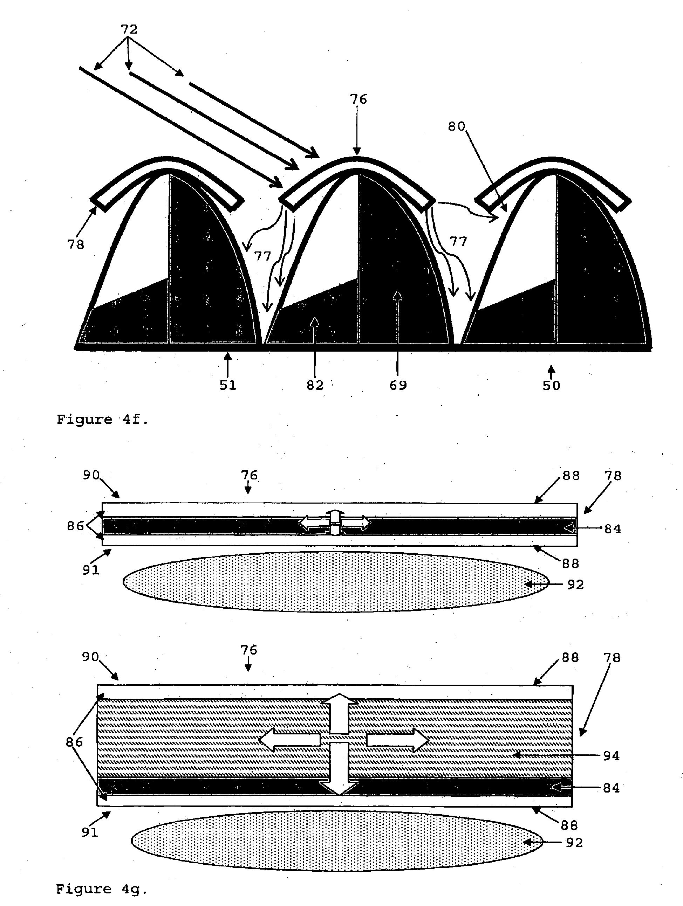

In one embodiment depicted in Figure 4, the bioreactor comprises a series of flexible plastic containments 50, such as tubes or triangular shaped bags, with no predetermined length other than that dictated by factors of practical engineering (Fig. 4a). The plastic containments 50 will sit side by side lying horizontally along the floor 51 of an open pond 52 with a uniform depth, deeper than conventional algae ponds with depths of only 30 cm to 50 cm. The containments 50 are filled with algae growth medium. The algae will be grown inside these plastic containments 50 while the inverted triangular spaces 54 between the containments will remain as clear water to allow the passage of light. As shown in Fig. 4b, the plastics of the containments 50 comprise the luminescent substrates 60 and will luminesce wavelengths of light useful for photosynthesis. Piping 56 with intermittent holes 58 for delivery of CO₂ rich gases will lie at the base 51 of the algae containment apparatuss 50 and produce a stream of gas bubbles to both infuse the growth medium with gaseous nutrients for photosynthetic growth as well as creating a scrubbing action, by the bubbles themselves, that will help to reduce bio-fouling on the face of the polymer materials. The gases will also form a convection type motion in the water to help mix the algae bringing them closer to and further away from the substrate surface, and therefore the light source. As is known in the field of algae biology, light and dark reactions in photosynthesis require the algae to spend a large portion of time away from the light source to prevent photo-inhibition.

The system can be designed as an open pond 52 or as a more dynamic raceway type design (see Figure 4c raceway design) that has proven to be the most effective open design to date. In a raceway design, the containments 50 would lie along the straight sections 67 while the curved sections 68 would be open to air as a place for infrastructure such as paddle wheels (or other devices for water circulation known in the art), harvesting equipment, water exchange, nutrient addition and gas exchange. In such a design, the water in the curved portions 68 may be filled with algae in open type design where it can be harvested. In this configuration the containments 50 are open at the ends to allow the algae to fill these sections. The clear water 54 between the containments 50 will be sealed off to prevent contamination of the clear water by algae. Alternatively, the curved sections 68 may be clear of algae where the clear spaces 54 extend into these open sections. The clear water should often be filtered to allow the best transmittance of light. In addition, the clear water will absorb a majority of the IR portion of the solar spectrum and will become warmer as a result. The clear water may be released and replaced to act as a thermostat for the entire system where excess heat is diverted away from the system. In winter months, the clear water may act as an insulator to keep the temperature of the algae above a set minimum according to the needs of the particular species.

The luminescent substrate 60 is surrounded by a layer of air 62 that creates a degree of total internal reflection to trap a portion of the luminescent light within the algae growth medium by the positioning of a boundary of higher index of refraction, i.e. water having an index of refraction of approximately 1.33, adjacent a boundary of a low index of refraction, i.e. the air with an index of refraction of approximately 1. This enables the algae containment apparatuss 50 to act as large light guides. The air layer 62 is maintained by a clear polymer sheet 64 outside of the luminescent substrate 60. It may also be advantageous to line the containment 50 with a thin, expendable polymer containment 66 to prevent fouling of the valuable substrate by direct contact with the algae biomass and thus increase the useful lifetime of the more expensive luminescent substrate.

When sunlight strikes the surface 53 of the pond 52 it will travel down the clear spaces 54 between the algae containment apparatuss 50 and either pass through the luminescent substrate 60 to directly illuminate the algae or first be absorbed by luminescent material disposed in the substrate and then be converted, by luminescence, to a lower wavelength of light useful for photosynthesis before being absorbed by the algal biomass. Whether sunlight is absorbed by the luminescent materials is largely dependent on the particular wavelengths of the light where certain wavelengths pass through the luminescent substrate 60, not lying within the major absorbance wavelengths of the disposed luminescent materials, while others do undergo an absorbance/emission process. The entire system of containments 50 can be oriented, according to cardinal directions, to lie north to south, east to west, or any direction in between. As shown in Fig. 4d, in either orientation, there will be a shadow 69 cast on one side of the containment 50 while the other side is illuminated 70 at times when the sun is not nearly or directly overhead. These periods of low angled sunlight represent the majority of daytime hours for all latitudes on earth. When sunlight 72 strikes the non-shaded side of the containment 50, the resulting luminescence from the luminescent substrate 60 will radiate partially outwards 74 and cause the shaded side 69 of the adjacent containment to be illuminated by useful wavelengths of light. These sides will alternate between shaded and illuminated between morning and evening hours as the sun moves across the sky.

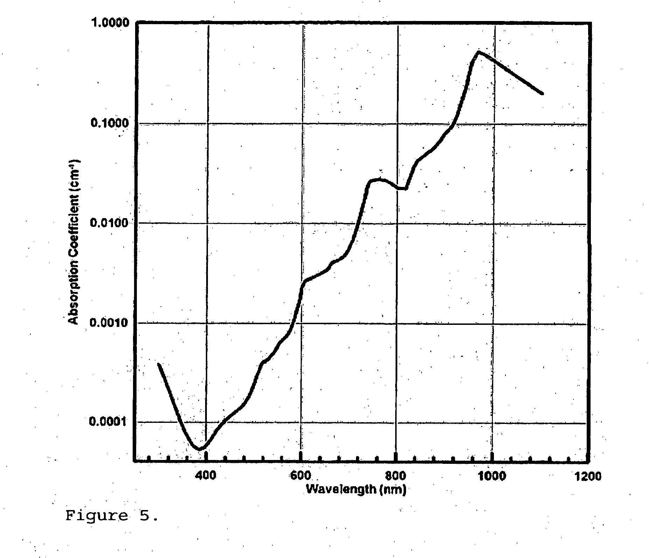

It is of particular importance to produce large amounts of red light between 600 nm and 700 nm as these wavelengths are used very efficiently by photosynthetic organisms. Also, in deeper pond systems, the absorbance by water of incoming red light can be a significant factor (Figure 5) where a clear channel of water will absorb approximately 90% of red light at 650 nm over a path of 2 meters. Shorter wavelengths of light such as orange, yellow, green and blue (575 nm to 400 nm) pass through water with much less loss and can be down-converted to red light at deeper depths after they have made their passage through the water column.

The shape of the system causes the effective algal surface to be more than doubled when compared to the surface area 53 of the pond 52 in which they are contained. The taller the containments 50 are made, the greater the effective area. This creates dilution of the sunlight striking the surface of the system over a larger area than it would encounter with a standard open pond. Thus, lower light intensities are encountered and therefore lowered occurrences of photo-inhibition are observed. In addition, the substrates that comprise the algae possess the ability to convert natural sunlight into a spectra of wavelengths more optimised for photosynthesis to achieve a higher solar energy conversion efficiency measured by an increase in biomass accumulation per square meter of area. This system may thus achieve higher algae concentrations as well as algae growth at greater depths. At greater concentrations of biomass, growth (e.g. biomass doubling) leads to exponentially greater gains. In addition, the higher biomass concentration allows for less energy to be expended in the harvesting system that begins with biomass concentration followed by dewatering.

As illustrated in Fig. 4e and Fig. 4f, a fluorescent light concentrator, or fluorescent light guide, 76 is added to the top of the algae containment apparatus 50. The addition of such a light guide must be weighed against its cost/benefit analysis that may be positive or negative depending on the additional biomass gained and its inherent value. The light guide 76 luminesces orange light 77 between 540 nm and 600 nm by the absorbance of light between 400 nm and 540 nm. Since this orange light 77 of higher energy wavelength is produced hear the surface of the water 53, the orange light will pass efficiently through the clear water channel 54, with minimal absorption losses, since its wavelengths are shorter than 600 nm. When the orange light 77 is produced it will be emitted out of the edges 78 of the light guide 76 that are bent slightly downwards to direct the light into the clear water channels 54.

Once the orange light 77 reaches the surface of the algae containment apparatuss 50 it will be largely absorbed by the luminescent materials disposed in the luminescent substrate 60 and further down-converted to a red light to enable efficient photosynthesis. To save on costs, the portion 80 of the containment 50 that lies underneath the light guide 76 may or may not be disposed with luminescent materials as it will receive readily optimised light as the light guide above filters the green wavelengths.

This light guide 76 will aid in illuminating the bottom portion 82 of an algae containment apparatus 50 that is deep (greater than 1 meter). In such a system, the bottom portion 82 of the containment 50, even when facing the direction of the sun, may remain shaded 69 at hours of low angled sunlight. The light guide 76 will work in concert with the containments 50 where the light guide converts green light to orange and the containment converts orange to red.

As depicted in Fig. 4g, the light guide 76 comprises a polymer sheet 84 embedded with a fluorescent dye to create a fluorescent solar collector where the luminescence is concentrated towards the edges 78 as is known in the art. Since the light guide 76 will be in contact with water it is therefore pertinent to surround the polymer sheet with a layer of air above and below 86 by the addition of two additional thin clear sheets of polymer 88. The light is trapped within the sheet by total internal reflection and conducted towards the edges 78 for all light that is within the critical angle defined by the refractive indices of the polymer and the surrounding air. For most plastics this angle is about 42 degrees from the vertical where all light that is less than this angle from the vertical is emitted out of the top 90 and bottom 91 faces of the sheet. For most applications the emission out of the faces 90, 91 is considered a loss, whereas it is an advantage of the present system that light emitted from the bottom face 91 of the sheet is transmitted directly into the algae biomass 92.

As with most fluorescent concentrators, there is significant loss due to reabsorption of fluoresced light when the emission wavelengths overlap somewhat with the absorbance due to the limited Stokes shift of most fluorescent dyes. The losses due to reabsorption become significant when the ratio of the thickness of the light guide is very small compared to that of the length that defines the pathway that the light must travel within the sheet to reach the edge. Apart from limiting the total length of the light guide to reduce collection efficiency losses, it also possible to increase the thickness of the light guide to increase the thickness to length ratio using an inexpensive material 94 with a similar index of refraction to the light guide polymer. This material may be a liquid or solid, but the most economic choices are liquids such as aqueous salt solutions (e.g. concentrated CaCl₂), or solvents such as DMSO, n-glycol (where n=1, 2, 3, 4 etc.), glycerine and other liquids or mixtures of liquids as is known in the art. Also, the layer containing the luminescent material 84 may also be a liquid such as synthetic liquids, paraffin oil or silicon based oils.

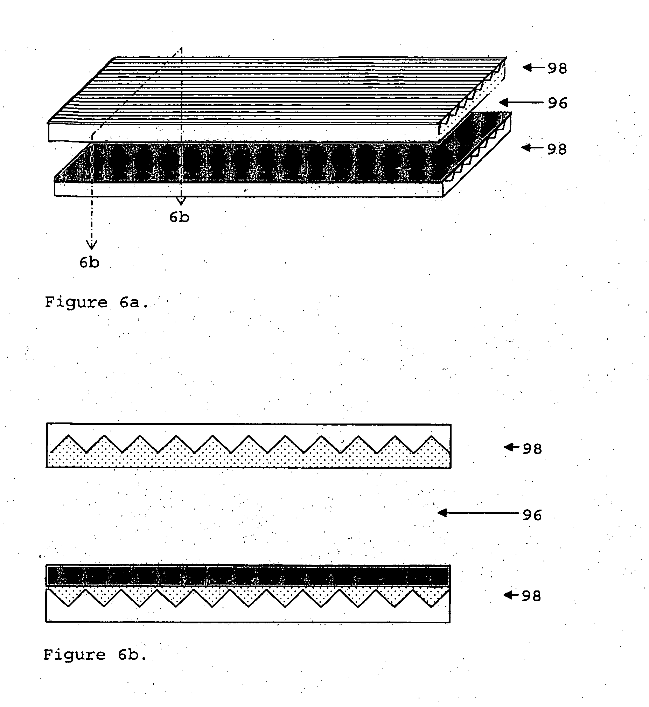

Other forms of light guides may be employed such as prismatic light guides (Figure 6) that are advantageous in allowing a large gap of air 96 to act as the light conduit where light spends very little time within the polymer materials 98. Such light guides are available commercially from companies such as 3M^{™}.

The substrates that comprise the algae containment apparatuss 50 are composed of a durable flexible polymer in which one or more luminescent materials are disposed. The luminescent material is in the form of a dissolved dye, e.g. an organic dye that absorbs a broad spectrum of light between 350 nm and 600nm and luminesces between 600 nm and 700 nm. This may also be achieved by a mixture of dyes that able the substrate to evenly collect a broader range of wavelengths. The substrate may also include a fluorescent material that absorbs UV and blue light to emit light between 400 nm and 475nm to satisfy the blue light requirements for photosynthesis. It is noted however that blue light can penetrate a water column with very little absorption losses such that blue light contained in the natural solar spectrum will reach the substrates even at deeper levels of the system without the addition of a blue emitting material. The ability to produce both red and blue emission from the substrate is useful in helping to illuminate adjacent containments that are shaded as described previously.

When mixing dyes in the same substrate layer there comes an inherent problem of re-absorption where high energy wavelengths of light that are produced from a blue emitting dye are absorbed by other dyes in the substrate rather than being emitted and used efficiently by the algae. To help alleviate this problem, diffusion particles are introduced to extract light out of the substrate giving the substrate a cloudy appearance rather than a translucent one. Such techniques are known in the art as light extraction techniques and are often achieved by the use of such particles as silicates and other small particles. Luminescent (fluorescent or phosphorescent) particles can achieve this diffusion of light while also serving in their original capacity as luminescent materials. Having phosphorescent materials in the substrates, such as a bright green emitting phosphor, will allow a portion of the UV and blue portions of the spectrum to be absorbed and released slowly over a period of many hours. This emission will extend into night-time hours and extend the effective growth hours of the system even after the sun has disappeared. While most phosphorescent materials emit blue and green light, there is a lack of red emitting phosphorescent materials with the same brightness and favourable properties as the more common blue-green counterparts. The more robust and strong emitting green phosphor is useful when disposed in a substrate with a red fluorescent dye. The green light created by the phosphor is then absorbed by the red emitting dye in the substrate creating a red glow in the absence of sunlight to produce the desired red light for algae growth.

## Claims

1. A bioreactor comprising a plurality of algae containment apparatuses, for growing an alga or a cyanobacterium, each algae containment apparatus comprising a first light guide, wherein:
the first light guide is a luminescent light guide comprising a first luminescent material and
the first light guide guides light to the algae containment apparatus underneath the first light guide; and
the first luminescent material emits visible light between 540 nm and 600 nm suitable for absorption by a second luminescent material; and
a second light guide is formed between adjacent algae containment apparatuses, wherein the algae containment apparatus underneath the first light guide comprises the second luminescent material that absorbs visible light between 540 nm and 600 nm emitted by the first luminescent material.

2. The bioreactor of Claim 1, wherein the second luminescent material emits red light.

3. The bioreactor of Claim 1 or Claim 2, wherein the second light guide is a liquid-filled light guide.

4. The bioreactor of Claim 3, wherein the liquid-filled light guide is filled with clear water.

5. The bioreactor of any one of Claims 1 to 4, wherein the algae containment apparatus further comprises a double wall that maintains a boundary for guiding light.

6. The bioreactor of any one of Claims 1 to 5, wherein the algae containment apparatus is triangular, with a base positioned away from a light source and a vertex positioned towards the light source.

7. The bioreactor of any one of Claims 1 to 6, wherein the algae containment apparatus is tubular.

8. The bioreactor of any one of Claims 1 to 7, wherein the algae containment apparatus is tubular and triangular, with a base positioned away from a light source and a vertex positioned towards the light source

9. The bioreactor of any one of Claims 1 to 8, wherein the first light guide reduces reabsorption of luminescence by the first luminescent material by increasing the thickness to length ratio of the first light guide using a material of similar refractive index to the first light guide, but lacking a luminescent material.

10. The bioreactor of any one of Claims 1 to 9, further comprising a bio-digester.

11. A method for growing an alga or a cyanobacterium comprising growing the alga or cyanobacterium in the bioreactor of any one of Claims 1 to 10.

## Patentansprüche

1. Bioreaktor, der eine Vielzahl von Algenbehältervorrichtungen umfasst, zum Züchten einer Alge oder eines Cyanobakteriums, wobei jede Algenbehältervorrichtung einen ersten Lichtleiter umfasst, wobei:
der erste Lichtleiter ein lumineszierender Lichtleiter ist, der ein erstes lumineszierendes Material umfasst; und
der erste Lichtleiter Licht in die Algenbehältervorrichtung unterhalb des ersten Lichtleiters führt; und
das erste lumineszierende Material sichtbares Licht zwischen 540 nm und 600 nm abgibt, das zur Absorption durch ein zweites lumineszierendes Material geeignet ist; und
ein zweiter Lichtleiter zwischen angrenzenden Algenbehältervorrichtungen gebildet wird, wobei die Algenbehältervorrichtung unterhalb des ersten Lichtleiters das zweite lumineszierende Material umfasst, das sichtbares Licht zwischen 540 nm und 600 nm absorbiert, das durch das erste lumineszierende Material abgegeben wird.

2. Bioreaktor nach Anspruch 1, wobei das zweite lumineszierende Material rotes Licht abgibt.

3. Bioreaktor nach Anspruch 1 oder Anspruch 2, wobei der zweite Lichtleiter ein flüssigkeitsgefüllter Lichtleiter ist.

4. Bioreaktor nach Anspruch 3, wobei der flüssigkeitsgefüllte Lichtleiter mit klarem Wasser gefüllt ist.

5. Bioreaktor nach einem der Ansprüche 1 bis 4, wobei die Algenbehältervorrichtung ferner eine doppelte Wand umfasst, die eine Grenze für leitendes Licht darstellt.

6. Bioreaktor nach einem der Ansprüche 1 bis 5, wobei die Algenbehältervorrichtung dreieckig ist, mit einer Basis, die von einer Lichtquelle abgewandt angeordnet ist und einem Scheitel, der in Richtung der Lichtquelle angeordnet ist.

7. Bioreaktor nach einem der Ansprüche 1 bis 6, wobei die Algenbehältervorrichtung röhrenförmig ist.

8. Bioreaktor nach einem der Ansprüche 1 bis 7, wobei die Algenbehältervorrichtung röhrenförmig und dreieckig ist, mit einer Basis, die von einer Lichtquelle abgewandt angeordnet ist und einem Scheitel, der in Richtung der Lichtquelle angeordnet ist.

9. Bioreaktor nach einem der Ansprüche 1 bis 8, wobei der erste Lichtleiter die Resorption von Lumineszenz durch das erste lumineszierende Material verringert durch Erhöhen des Dicke zu Länge Verhältnisses des ersten Lichtleiters, unter Verwendung eines Materials mit ähnlicher Brechzahl wie der erste Lichtleiter, jedoch ohne ein lumineszierendes Material.

10. Bioreaktor nach einem der Ansprüche 1 bis 9, der ferner einen Biofermenter umfasst.

11. Verfahren zum Züchten einer Alge oder eines Cyanobakteriums, welches das Züchten der Alge oder des Cyanobakteriums in dem Bioreaktor nach einem der Ansprüche 1 bis 10 umfasst.

## Revendications

1. Bioréacteur comprenant une pluralité d'appareils de confinement d'algues, pour faire pousser une algue ou une cyanobactérie, chaque appareil de confinement d'algues comprenant un premier guide de lumière, dans lequel :
le premier guide de lumière est un guide de lumière luminescente comprenant un premier matériau luminescent ; et
le premier guide de lumière guide la lumière jusqu'à l'appareil de confinement d'algues au-dessous du premier guide de lumière ; et
le premier matériau luminescent émet de la lumière visible entre 540 nm et 600 nm approprié pour l'absorption par un second matériau luminescent ; et
un second guide de lumière est formé entre des appareils de confinement d'algues adjacents, dans lequel l'appareil de confinement d'algues au-dessous du premier guide de lumière comprend le second matériau luminescent qui absorbe la lumière visible entre 540 nm et 600 nm émise par le premier matériau luminescent.

2. Bioréacteur selon la revendication 1, dans lequel le second matériau luminescent émet de la lumière rouge.

3. Bioréacteur selon la revendication 1 ou la revendication 2, dans lequel le second guide de lumière est un guide de lumière rempli de liquide.

4. Bioréacteur selon la revendication 3, dans lequel le guide de lumière rempli de liquide est rempli avec de l'eau potable.

5. Bioréacteur selon l'une quelconque des revendications 1 à 4, dans lequel l'appareil de confinement d'algues comprend en outre une double paroi qui maintient une limite pour guider la lumière.

6. Bioréacteur selon l'une quelconque des revendications 1 à 5, dans lequel l'appareil de confinement d'algues est triangulaire, avec une base positionnée à distance d'une source de lumière et un sommet positionné vers la source de lumière.

7. Bioréacteur selon l'une quelconque des revendications 1 à 6, dans lequel l'appareil de confinement d'algues est tubulaire.

8. Bioréacteur selon l'une quelconque des revendications 1 à 7, dans lequel l'appareil de confinement d'algues est tubulaire et triangulaire, avec une base positionnée à distance d'une source de lumière et un sommet positionné vers la source de lumière.

9. Bioréacteur selon l'une quelconque des revendications 1 à 8, dans lequel le premier guide de lumière réduit la réabsorption de luminescence par le premier matériau luminescent en augmentant le rapport d'épaisseur sur longueur du premier guide de lumière en utilisant un matériau d'indice de réfraction similaire au premier guide de lumière, mais en l'absence d'un matériau luminescent.

10. Bioréacteur selon l'une quelconque des revendications 1 à 9, comprenant en outre un biodigesteur.

11. Procédé pour faire pousser une algue ou une cyanobactérie comprenant l'étape consistant à faire pousser l'algue ou la cyanobactérie dans le bioréacteur selon l'une quelconque des revendications 1 à 10.
